# EUROPEAN PATENT APPLICATION

(11) **EP 4 023 632 A1**
(43) Date of publication of application: **06.07.2022**
(21) Application number: 21870542.4
(22) Date of filing: 29.10.2021
(51) Int. Cl.: C07C 249/04, C07C 251/38, C07C 251/44, B01J 8/00

(54) **METHOD FOR INTEGRATING AMMOXIMATION REACTION AND SEPARATION AND DEVICE THEREOF**

(30) Priority: 02.11.2020 CN 202011205261
(71) Applicant: Hubei Jinxiangning Chemical Engineering Techenology Co., Ltd, Wuhan, Hubei 430000 (CN)
(72) Inventor: WU, Jian, Wuhan Hubei 430000 (CN); ZHAO, Chengjun, Wuhan Hubei 430000 (CN); WEI, Tianrong, Wuhan Hubei 430000 (CN); ZHOU, Xuejun, Wuhan Hubei 430000 (CN); LI, Wei, Wuhan Hubei 430000 (CN); CHE, Xiaojun, Wuhan Hubei 430000 (CN); LI, Hui, Wuhan Hubei 430000 (CN)
(74) Representative: Sach, Greg Robert
(86) International application number: PCT/CN2021/127385
(87) International publication number: WO 2022/073525

(57) **Abstract**

The present invention discloses a method for integrating ammoximation reaction and separation. In the present invention, a titanium silicalite molecular sieve is used as a catalyst, a ketone, hydrogen peroxide and ammonia are reacted by ammoximation reaction to form an oxime, the reaction materials are filtered by cross-flow filtration to separate clear liquid, a portion thereof is then withdrawn and an inert alkane solvent is added to extract the oxime, and turbid liquid containing the catalyst is recycled back to the reaction system. The method for integrating reaction and separation can reduce the filtration load of cross-flow membrane, decrease the consumption of the catalyst, save investment and operation costs and has good industrial application value.

## Description

### TECHNICAL FIELD

The present invention relates to a method for integrating ammoximation reaction and separation, in particular a method and a device for ammoximation reaction and separation of its products oxime, water and catalyst.

### BACKGROUND

Titanosilicate materials can catalyze the ammoximation reaction of aliphatic ketones or aromatic ketones with hydrogen peroxide and ammonia to form the corresponding oximes, and then a series of amides can be synthesized through Beckmann rearrangement. Caprolactam, scientific name ε-caprolactam, is an important petrochemical product and widely used in the manufacture of chinlon and engineering plastics. Therefore, the preparation of cyclohexanone oxime by ammoximation of cyclohexanone and the rearrangement of cyclohexanone oxime to produce caprolactam has important application value, at present, cyclohexanone-hydroxylamine method, cyclohexane photonitrosation method and toluene method have been gradually replaced and it becomes a mainstream method for industrial production of caprolactam.

In 1988, US Patent No. 4745221 for the first time discloses a method for forming cyclohexanone oxime through ammoximation of cyclohexanone, hydrogen peroxide and ammonia in the liquid phase: cyclohexanone selectivity is 98. 2% under mild conditions of 60°C~80°C using TS-1 as a catalyst and hydrogen peroxide as oxygen source. This method has a short process flow, avoids the complicated synthesis process of hydroxylamine and the generation of SOx or NOx, and does not produce ammonium sulfate by-product and is environmentally friendly. Subsequently, US Patent No. 4894478 discloses a multi-step liquid phase catalysis method to divide the oximation process of cyclohexanone into two steps, thereby improving the yield of cyclohexanone oxime.

In order to solve the problems of catalyst separation and efficiency in the amidoximation process, CN 1191125 and CN 1234683 disclose a method for separating titanium silicalite molecular sieves in a ketone ammoximation reaction system respectively, and in the ketone ammoximation reaction system of a water-miscible low-carbon alcohol and an excess of aqueous ammonia at a concentration of 30%-50% (mass fraction), materials comprising the reaction product oxime and the titanium silicalite molecular sieves are placed to settle in a settler at a temperature of 60-85°C and a linear velocity of 0.5-3 cm/min for not less than 10 minutes. This method can solve the problem of separation of catalyst and reaction product and improve the utilization rate of molecular sieves.

Due to small particle size of titanium-silicon molecular sieves, the method of settlement separation is not suitable for large-scale industrial production, and the separation efficiency needs to be improved. CN 100586928 discloses a method for preparing cyclohexanone oxime, which is characterized in that the method is continuously circulated in a loop reactor, subjecting a reaction slurry comprising a catalyst, ammonia, cyclohexanone, hydrogen peroxide and tert-butanol solvent to an ammoximation reaction in a loop, then performing solid-liquid separation on the reaction slurry in a filtration element in a solid-liquid separation assembly in a cross-flow manner, the obtained clear liquid flows out in the radial direction of the filtration element and is recovered, and the concentrated slurry is continuously circulated in the loop.

The above-mentioned ammoximation reaction and separation methods are all carried out in a system in which tert-butanol is a solvent. A water-miscible low-carbon alcohol (such as tert-butanol) is used as a solvent in order to improve the solubility of the ammoximation product in the system, so as to facilitate the reaction. However, the process thus designed will bring a number of disadvantages. Firstly, the low-carbon alcohol as a reaction solvent accounts for a large proportion in the reaction liquid, which increases the separation load of the reaction product and the catalyst and seriously affects the flux of cross-flow filtration. Secondly, because the solvent can not be stably present in the oleum system of the subsequent Beckmann rearrangement reaction, and after multiple separation processes such as distillation and extraction, the process flow is complicated, the energy consumption is large, and the stability of the oxime is relatively poor and trace impurities are generated in the subsequent rearrangement, which brings difficulties to the purification process.

CN 100386307 discloses a method for preparing amides by oximation and rearrangement using aliphatic or/and cycloaliphatic ketones as raw materials: in the presence of an inert solvent, a ketone, hydrogen peroxide and ammonia are catalyzed to form an oxime solution, and the Beckmann rearrangement of the oil phase product occurs under the action of oleum, followed by hydrolysis and neutralization to obtain amides. CN 1939897 discloses a method for separating and preparing a ketoxime by three-phase ammoximation reaction, wherein a ketone, hydrogen peroxide and ammonia are subjected to liquid-liquid-solid three-phase heterogeneous oximation reaction in the presence of a solid catalyst and a water-insoluble or slightly soluble solvent to form an oxime; after phase separation of the oximation reaction product, the light phase is a solvent phase containing oxime, and a part of water is directly separated from the heavy phase and then recycled; alternatively, the heavy phase may be extracted with a water-insoluble or slightly soluble solvent to extract the oxime therefrom, and a part of water may be separated from the heavy phase raffinate containing the catalyst and then recycled.

CN 105837468 discloses a method for preparing cyclohexanone oxime: cyclohexanone, ammonia and hydrogen peroxide are subjected to ammoximation reaction in a solvent in the presence of an oximation catalyst, wherein the solvent is an aqueous solution containing a small amount of an inert organic solvent, ammoximation can obtain high cyclohexanone conversion and cyclohexanone oxime selectivity, and ammoximation can obtain high cyclohexanone conversion rate and cyclohexanone oxime selectivity. CN 104926689 discloses a solvent-free method for preparing cyclohexanone oxime, and the specific preparation comprises the preparation of a reaction system, oximation reaction and separation of products. The product can be directly separated from the reaction solution by condensation, and the energy consumption is low, the preparation process is environmentally friendly, and the industrialized implementation is easy.

### SUMMARY

The present inventionprovides a method and a device for ammoximation reaction and the separation of its products oxime, water and catalyst. It is a purpose of the present invention to provide a method and device for continuous and efficient separation of product and catalyst for a solvent-free system ammoximation or an aqueous system ammoximation process containing a small amount of an inert organic solvent.

The purpose of the present invention is to be achieved in the following mode:

a method for integrating ammoximation reaction and separation, comprising following four steps:
(1) reacting a ketone, hydrogen peroxide and ammonia to form an oxime in the presence of a titanium silicalite molecular sieve as a catalyst;
(2) separating clear liquid containing water and the oxime from reaction materials by cross-flow filtration;
(3) subjecting a portion of the materials after separation of the clear liquid to mixed extraction before being recycled back to a reactor, and adding an inert alkane solvent to extract so as to obtain an organic phase which is an oxime solution; and
(4) returning extracted aqueous phase turbid liquid containing the catalyst to an ammoximation reaction system.

Further, the ammoximation reaction of a ketone, hydrogen peroxide and ammonia is carried out using a titanium silicalite molecular sieve as a catalyst, and the titanium silicalite molecular sieve is one or more of Ti-MWW, TS-1, TS-2, Ti-β, Ti-SBA-15, Ti-MCM-41 or Ti-MOR, preferably one of Ti-MWW, TS-1 or a mixture of both, particularly preferably Ti-MWW

Further, the ketone of one of the raw materials for the ammoximation reaction is an aliphatic ketone with 3~10 carbon atoms, or a cycloaliphatic ketone or an aromatic ketone with 5~10 carbon atoms; a concentration of hydrogen peroxide used is 10%-80% by a mass fraction, preferably 25%~60%; and a molar ratio of the ketone, hydrogen peroxide to ammonia is 1: 1.0~2: 1.0~4, preferably 1: 1.0~1.2: 1.0~2.0.

Further, the ammoximation reaction of a ketone, hydrogen peroxide and ammonia is performed under a condition of no solvent or a small amount of an inert alkane solvent, wherein reaction temperature is 40~100 °C, and reaction pressure is 0.1~1 MPa, and the reaction pressure controlled finally does not exceed 1 Mpa.

Further, a device for integrating ammoximation reaction and separation comprises a tank reactor, a cross-flow filter and a mixed extraction unit, wherein the tank reactor, the cross-flow filter and the mixed extraction equipment are successively connected;
a discharge end of materials of the cross-flow filter is in communication with the tank reactor via a material reflux pipe;
a discharge end of aqueous phase of the mixed extraction unit is in communication with the tank reactor via an aqueous phase reflux pipe; and
the mixed extraction unit is connected to an inert alkane solvent feed pipe.

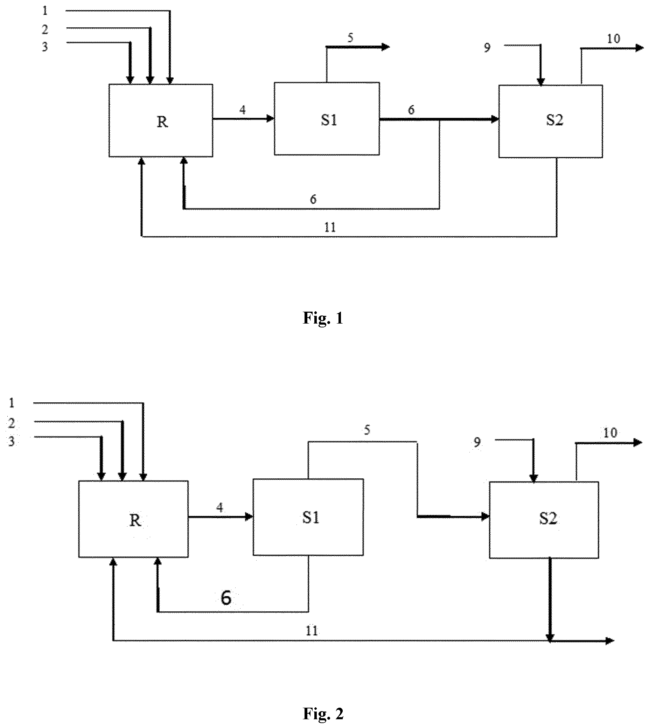

The method for integrating an ammoximation reaction and separation provided by the present invention comprises the steps such as an ammoximation reaction, cross-flow membrane filtration separation, extraction separation, which will be described below with reference to Fig. 1.

The ammoximation reaction is based on ketone, hydrogen peroxide and ammonia as raw materials under a condition of no solvent or a small amount of an inert alkane solvent through the catalytic reaction of titanium silicon molecular sieve to form an oxime and water. The ammoximation reaction is performed in a tank reactor R and a circulation pipeline, wherein the circulation pipeline is provided with a cross-flow filter S1, and the reaction product is firstly filtered through the cross-flow filter S1 to separate out clear liquid; a portion of the materials is subjected to mixed extraction unit S2 before being recycled back to a reactor R, and an inert alkane solvent is added to extract so as to obtain an organic phase which is an oxime solution, and the aqueous phase is a catalyst-enriched aqueous solution and is returned to the reactor R via the circulation pipeline.

The above-mentioned cross-flow filter S1 is a metal membrane pipe or a ceramic membrane pipe, and the filtration accuracy is 20 nm~2000 nm.

Under a very wide range of reaction conditions and membrane pipe standards, the cross-flow filter S1 can intercept catalyst particles in the reaction materials, and a liquid level of the ammoximation reactor can be maintained constant by controlling a flow rate of the cross-flow filtration clear liquid, and a mass fraction of oxime in the cross-flow filtration clear liquid is 0.01%-50%.

A portion of the materials after separation of the clear liquid is withdrawn and mixed with the inert alkane solvent 9 in the mixed extraction S2 unit before being recycled back to the reactor R to extract the oxime in the reaction materials. The mixed extraction S2 unit may be one or a combination of two or more of a mixer, a pipe or a cyclone equipment. A ratio of a flow rate of materials withdrawn into the mixed extraction S2 unit to a flow rate of materials recycled back to the reactor R is 0.01~0.3: 1.

The inert alkane solvent is an alkane or cycloalkane or aromatic hydrocarbon with 4~10 carbon atoms or a mixture thereof which is insoluble or slightly soluble with water. Since the solubility of oximes in inert alkanes is much greater than their solubility in water, it is easy to obtain oxime-inert alkane solution, which is very suitable for Beckmann rearrangement reactions in a solvent vaporization heat transfer mode. A ratio of a flow rate of the inert alkane solvent to materials into S2 unit to perform the mixed extraction is 0.1~10: 1. This ratio will determine the concentration of the oxime-inert alkane solution and can be chosen according to the requirements of the subsequent Beckmann rearrangement reaction.

The aqueous phase 11 obtained in the mixed extraction S2 unit is turbid liquid containing the catalyst, which is recycled back to the ammoximation reactor, wherein the mass fraction of the catalyst is between 1%-10%.

As can be seen with reference to Fig. 1, in the method for integrating ammoximation and separation provided by the present invention, the separation of water, product oxime and catalyst is achieved simultaneously by the ammoximation performed in the reactor R and the circulation pipeline and the combination of the cross-flow filter S1 and the mixed extraction unit S2.

The invention provides a method for integrating ammoximation reaction and separation, which has the following advantageous effects:
(1) The traditional ammoximation process uses low-carbon alcohol as the solvent, and a membrane is provided inside the reactor to separate or a cross-flow membrane is provided outside the reactor to separate the product and the catalyst, due to the simultaneous separation of the reaction product oxime, water, solvent and catalyst, the membrane flux is easily insufficient, while in the present invention, the ammoximation reaction is performed under the condition of no solvent or a small amount of an inert alkane solvent, and the water, the product oxime and the catalyst are separated with high selectivity through a combination of a cross-flow filter and a mixed extraction unit, which can greatly reduce the load of membrane separation and save equipment investment.
(2) The method for integrating ammoximation reaction and separation used in the present invention can minimize the consumption of catalyst in the ammoximation reaction with no solvent or a small amount of inert alkane solvent, and save the operation cost.
(3) Due to the cancellation of the low-carbon alcohol solvent, the production capacity of the oximation reactor can be greatly improved, and the number of reactors can be reduced under the requirement of the same production capacity, which further saves equipment investment.
(4) In a mixed extraction unit outside the circulation system, due to the large difference between the density of the inert alkane and the reaction materials, the rapid and efficient separation of the product oxime and the turbid liquid containing the catalyst can be achieved. In particular, the concentration of the oxime-inert alkane solution thus obtained can be adjusted according to the needs of the subsequent Beckmann rearrangement reaction without affecting the ammoximation reaction and separation, and the process operation is simple and flexible.

In summary, the method for integrating the ammoximation reaction and separation is provided by the present invention, the continuous and efficient separation of product oxime, water and catalyst can be achieved on the premise of obtaining high cyclohexanone conversion rate and cyclohexanone oxime selectivity, greatly saving equipment investment and improving device production capacity. The oxime-inert alkane solution thus obtained is very suitable for Beckmann rearrangement reaction of solvent vaporization and heat transfer, which abandons the subsequent complicated separation and purification brought by the traditional ammoximation process using low-carbon alcohol as solvent, and significantly saves the investment and energy consumption of ammoximation and rearrangement, and has great industrial application value.

### BRIEF DESCRIPTION OF THE DRAWINGS

Fig. 1 is a schematic diagram of the present invention.
Fig. 2 is a schematic diagram of Comparative Embodiment 1 in comparison with the present invention.
Fig. 3 is a schematic diagram of Comparative Embodiment 2 in comparison with the present invention.
Fig. 4 is a schematic diagram of the device of the present invention.

In the Fig.s, ketone 1, hydrogen peroxide 2, ammonia 3, reaction product 4, clear liquid 5, materials 6, an inert alkane solvent 9, an organic phase 10, an aqueous phase 11, a tank reactor R, a cross-flow filter S1, a mixed extraction unit S2.

### DETAILED DESCRIPTION OF THE EMBODIMENTS

The present invention will be further described below through specific embodiments, it should be noted that the following embodiments are illustrative only and the content of the present invention is not limited thereto.

Embodiment 1: as shown in Fig. 1, cyclohexanone (ketone 1), ammonia 3, and hydrogen peroxide 2 were respectively measured by a mass flow meter and then continuously added to an ammoximation reactor (a tank reactor R) containing a Ti-MWW catalyst. The flow rate of cyclohexanone ketone 1 was 140 kg/h, the flow rate of hydrogen peroxide 2 was 180 kg/h with the concentration of 27.5%, and the flow rate of ammonia gas was 32 kg/h. The effective volume of the oximation reactor was 1.0 m³, the reaction temperature was 80°C and the circulation flow rate was 11 m³/h. The circulation pipeline was equipped with a ceramic cross-flow filter (a cross-flow filter S1); after the reaction product 4 was filtered out clear liquid 5 through the cross-flow filter SI, the materials 6 were withdrawn at a flow rate of 1.5 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 1000 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor. According to the above-mentioned process conditions, continuous and stable operation was carried out for 720 hours, sampling and analysis were performed to calculate the conversion rate and selectivity, and the consumption was calculated by the catalyst concentration, the results are shown in Table 1.

Comparative Embodiment 1: feed flow rate, ratio, reaction temperature and circulation amount of the ammoximation reaction were the same as those in Embodiment 1. As shown in Fig. 2, the difference was that clear liquid 5 filtered out by the cross-flow membrane filter S1 entered the cyclone extractor (the mixed extraction unit S2) and the materials 6 were recycled back to the ammoximation reactor. Cyclohexane (an inert alkane solvent 9) was added to the cyclone extractor (the mixed extraction unit S2) at a flow rate of 1000 kg/h, and the light phase (the organic phase 10) was separated out as a cyclohexane-oxime solution, which entered the subsequent rearrangement reactor. A small portion of the heavy phase (the aqueous phase 11) separated out by the cyclone extractor was discharged from the system to maintain the liquid level of the ammoximation reactor constant, and most of the remaining water phase 11 was recycled back to the ammoximation reactor R. According to the above-mentioned process conditions, continuous and stable operation was carried out for 720 hours, sampling and analysis were performed to calculate the conversion rate and selectivity, and the consumption was calculated by the catalyst concentration, the results are shown in Table 1.

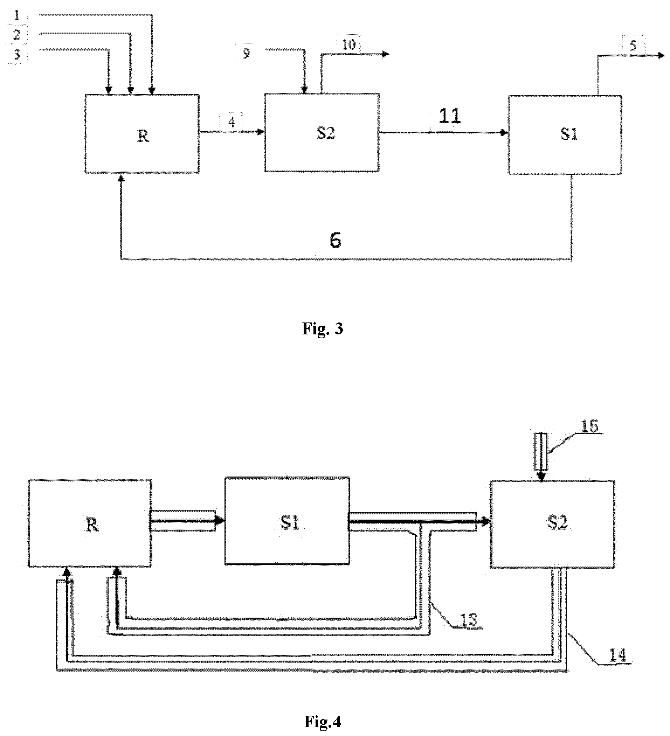

Comparative Embodiment 2: feed flow rate, ratio, reaction temperature and circulation amount of the ammoximation reaction were the same as those in Embodiment 1. As shown in Fig. 3, the difference was that the sequence of cross-flow membrane filter S1 and cyclone extractor S2 was changed, the reaction product 4 firstly passed through the cyclone extractor (the mixed extraction unit S2), cyclohexane (an inert alkane solvent 9) was added to the cyclone extractor (the mixed extraction unit S2) at a flow rate of 1000 kg/h, and the light phase 10 was separated out as a cyclohexane-oxime solution, which entered the subsequent rearrangement reactor. The heavy phase (the aqueous phase 11) separated out by the cyclone extractor (the mixed extraction unit S2) entered the clear liquid 5 filtered out by the cross-flow membrane filter S1 to maintain the liquid level of the ammoximation reactor constant and the materials 6 were recycled back to the ammoximation reactor (the tank reactor R). According to the above-mentioned process conditions, continuous and stable operation was carried out for 720 hours, sampling and analysis were performed to calculate the conversion rate and selectivity, and the consumption was calculated by the catalyst concentration, the results are shown in Table 1.

**Table 1 Comparison of results of different separation methods**

| Experiment No. | Cyclohexanone conversion rate % | Cyclohexanone oxime selectivity % | Required membrane area m² | Catalyst consumption kg/t oxime |
|---|---|---|---|---|
| Embodiment 1 | 99.5 | 99.4 | 1.0 | 0.33 |
| Comparative Embodiment 1 | 98.4 | 98.8 | 6.0 | 0.60 |
| Comparative Embodiment 2 | 98.2 | 98.7 | 1.0 | 0.80 |

As can be seen from a comparison of the results of Embodiment 1 with those of Comparative Embodiment 1 and Comparative Embodiment 2, using the method for integrating the ammoximation reaction and separation of the present invention, the stable and continuous operation of the process can be ensured with less membrane filtration area, and the catalyst The consumption was the lowest and the cyclohexanone conversion and cyclohexanone oxime selectivity were the highest.

Embodiment 2, cyclohexanone (ketone 1), ammonia 3, and hydrogen peroxide 2 were respectively measured by a mass flow meter and then continuously added to an ammoximation reactor (a tank reactor R) containing a Ti-MOR catalyst. The flow rate of cyclohexanone (ketone 1) was 200-240 kg/h, the flow rate of hydrogen peroxide 2 was 350-410 kg/h with the concentration of 26.5-28%, and the flow rate of ammonia gas was 49-70 kg/h. The effective volume of the oximation reactor was 1.5-2.5 m³, the reaction temperature was 80-88°C and the circulation flow rate was 10-11.5 m³/h. The circulation pipeline was equipped with a ceramic cross-flow filter (a cross-flow filter S1); after the reaction product 4 was filtered out clear liquid 5 through the cross-flow filter SI, the materials 6 were withdrawn at a flow rate of 1.2-1.8 m³/h into a cyclone extractor (a mixed extraction unit S2), and the rest was recycled back to the ammoximation reactor (the tank reactor R). N-hexane (an inert alkane solvent 9) was added to the cyclone extractor (the mixed extraction unit S2) at a flow rate of 950-2100 kg/h, and the light phase (the organic phase 10) N-hexane was separated out as an oxime solution, which entered the subsequent rearrangement reactor. The heavy phase (the aqueous phase 11) separated from the cyclone extractor (the mixed extraction unit S2) was recycled back to the ammoximation reactor.

Embodiment 3, cyclohexanone (ketone 1), ammonia 3, and hydrogen peroxide 2 were respectively measured by a mass flow meter and then continuously added to an amidoximation reactor (a tank reactor R) containing catalysts TS-1 and TS-2. The flow rate of cyclohexanone (ketone 1) was 130-420 kg/h, the flow rate of hydrogen peroxide 2 was 160-750 kg/h with the concentration of 25-29%, and the flow rate of ammonia gas was 25-145 kg/h. The effective volume of the oximation reactor was 0.8-3.5 m³, the reaction temperature was 80-90°C and the circulation flow rate was 10-19 m³/h. The circulation pipeline was equipped with a ceramic cross-flow filter (a cross-flow filter S1), after the reaction product 4 was filtered out clear liquid 5 through the cross-flow filter SI, the materials 6 were withdrawn at a flow rate of 1.3-3.2 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 850-3500 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 4, cyclohexanone (ketone 1), ammonia 3, and hydrogen peroxide 2 were respectively measured by a mass flow meter and then continuously added to an amidoximation reactor (a tank reactor R) containing catalysts TS-1 and TS-2. The flow rate of cyclohexanone (ketone 1) was 50-200 kg/h, the flow rate of hydrogen peroxide 2 was 60-340 kg/h with the concentration of 27-33%, and the flow rate of ammonia gas was 19-48 kg/h. The effective volume of the oximation reactor was 0.4-1.3 m³, the reaction temperature was 80-90°C and the circulation flow rate was 8-13 m³/h. The circulation pipeline was equipped with a metal membrane pipe filtration device (a cross-flow filter S1); after the reaction product 4 was filtered out clear liquid 5 through the cross-flow filter SI, the materials 6 were withdrawn at a flow rate of 1.2-1.8 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 750-1500 kg/h, and the light phase (the organic phase 10) was separated as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 5, cyclohexanone (ketone 1), ammonia 3, and hydrogen peroxide 2 were respectively measured by a mass flow meter and then continuously added to an amidoximation reactor (a tank reactor R) containing a Ti-MCM-41 catalyst. The flow rate of cyclohexanone (ketone 1) was 20-48 kg/h, the flow rate of hydrogen peroxide 2 was 30-95 kg/h with the concentration of 26-31%, and the flow rate of ammonia gas was 5-11 kg/h. The effective volume of the oximation reactor was 0.2-0.8 m³, the reaction temperature was 76-85°C and the circulation flow rate was 3-4.5 m³/h. The circulation pipeline was equipped with a metal membrane pipe filtration device (a cross-flow filter S1); after the reaction product 4 was filtered out clear liquid 5 through the cross-flow filter SI, the materials 6 were withdrawn at a flow rate of 0.4-0.6 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 120-410 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 6, cyclohexanone (ketone 1), ammonia 3, and hydrogen peroxide 2 were respectively measured by a mass flow meter and then continuously added to an amidoximation reactor (a tank reactor R) containing a Ti-SBA-15 catalyst. The flow rate of cyclohexanone (ketone 1) was 300-600 kg/h, the flow rate of hydrogen peroxide 2 was 320-1090 kg/h with the concentration of 27.5-30%, and the flow rate of ammonia was 50-130 kg/h. The effective volume of the oximation reactor was 1.5-5 m³, the reaction temperature is 79-90°C and the circulation flow rate was 16.5-28 m³/h. The circulation pipeline was equipped with a metal membrane pipe filtration device (a cross-flow filter S1); after the reaction product 4 was filtered out clear liquid 5 through the cross-flow filter SI, the materials 6 were withdrawn at a flow rate of 2.1-4.8 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 1500-3900 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 7, cyclohexanone (ketone 1), ammonia 3, and hydrogen peroxide 2 were respectively measured by a mass flow meter and then continuously added to an amidoximation reactor (a tank reactor R) containing TS-2 and Ti-βcatalyst. The flow rate of cyclohexanone (ketone 1) was 610-800 kg/h, the flow rate of hydrogen peroxide 2 was 1100-1590 kg/h with the concentration of 27-31%, and the flow rate of ammonia gas was 120-160 kg/h. The effective volume of the oximation reactor was 3.0-6.5 m³, the reaction temperature was 79-90°C and the circulation flow rate was 23-36 m³/h. The circulation pipeline was equipped with a metal membrane pipe filtration device (a cross-flow filter S1); after the reaction product 4 was filtered out clear liquid 5 through the cross-flow filter SI, the materials 6 were withdrawn at a flow rate of 2.5-6.5 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 2100-4600 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 8, cyclohexanone (ketone 1), ammonia 3, and hydrogen peroxide 2 were respectively measured by a mass flow meter and then continuously added to an amidoximation reactor (a tank reactor R) containing a Ti-SBA-15 catalyst. The flow rate of cyclohexanone (ketone 1) was 810-900 kg/h, the flow rate of hydrogen peroxide 2 was 1300-1800 kg/h with the concentration of 27-31%, and the flow rate of ammonia gas was 32 kg/h. The effective volume of the oximation reactor was 3.0-7.5 m³, the reaction temperature was 81°Cand the circulation flow rate was 28-42 m³/h. The circulation pipeline was equipped with a metal membrane pipe filtration device (a cross-flow filter S1); after the reaction product 4 was filtered out clear liquid 5 through the cross-flow filter SI, the materials 6 were withdrawn at a flow rate of 2.8-7.5 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 3020-5200 kg/h, and the light phase (the organic phase 10) was separated out as a cyclohexane-oxime solution, which entered the subsequent rearrangement reactor. The heavy phase (aqueous phase 11) separated from the cyclone extractor (the mixed extraction unit S2) was recycled back to the ammoximation reactor.

Embodiment 9, cyclohexanone (ketone 1), ammonia 3, and hydrogen peroxide 2 were respectively measured by a mass flow meter and then continuously added to an amidoximation reactor (a tank reactor R) containing a Ti-SBA-15 catalyst. The flow rate of cyclohexanone (ketone 1) was 110-190 kg/h, the flow rate of hydrogen peroxide 2 was 230-270 kg/h with the concentration of 31.5-36%, and the flow rate of ammonia gas was 28-41 kg/h. The effective volume of the oximation reactor was 0.7-1.3 m³, the reaction temperature was 70-95°C and the circulation flow rate was 10-12 m³/h. The circulation pipeline was equipped with a metal membrane pipe filtration device (a cross-flow filter S1); after the reaction product 4 was filtered out clear liquid 5 through the cross-flow filter S1, the materials 6 were withdrawn at a flow rate of 1.7 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 900-1100 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 10, cyclohexanone (ketone 1), ammonia 3, and hydrogen peroxide 2 were respectively by a mass flow meter and then continuously added to an ammoximation reactor (a tank reactor R) containing a Ti-MWW catalyst. The flow rate of cyclohexanone (ketone 1) was 110-190 kg/h, the flow rate of hydrogen peroxide 2 was 120-290 kg/h with the concentration of 28.5-32%, and the flow rate of ammonia gas was 29-51 kg/h. The effective volume of the oximation reactor was 0.8-1.6 m³, the reaction temperature was 75-89°C and the circulation flow rate was 9-13 m³/h. The circulation pipeline was equipped with a metal membrane pipe filtration device (a cross-flow filter S1); after the reaction product 4 was filtered out clear liquid 5 through the cross-flow filter SI, the materials 6 were withdrawn at a flow rate of 1-2.1 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 800-1200 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 11, cyclohexanone (ketone 1), ammonia 3, and hydrogen peroxide 2 were respectively measured by a mass flow meter and then continuously added to an amidoximation reactor (a tank reactor R) containing TS-1, TS-2, and Ti-βcatalyst. The flow rate of cyclohexanone (ketone 1) was 120-180 kg/h, the flow rate of hydrogen peroxide 2 was 205 kg/h with the concentration of 36. 5%, and the flow rate of ammonia gas was 34 kg/h. The effective volume of the oximation reactor was 0.8-1.9 m³, the reaction temperature was 80-92°C and the circulation flow rate was 10-11.5 m³/h. The circulation pipeline was equipped with a metal membrane pipe filtration device (a cross-flow filter S1); after the reaction product 4 was filtered out clear liquid 5 through the cross-flow filter SI, the materials 6 were withdrawn at a flow rate of 1.4-1.55 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 920-1130 kg/h, and the light phase (organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 12, cyclohexanone (ketone 1), ammonia 3, and hydrogen peroxide 2 were respectively measured by a mass flow meter and then continuously added to an ammoximation reactor (a tank reactor R) containing a Ti-MWW catalyst. The flow rate of cyclohexanone (ketone 1) was 130-180 kg/h, the flow rate of hydrogen peroxide 2 was 140-230 kg/h with the concentration of 31-39.5%, and the flow rate of ammonia gas was 29-36 kg/h. The effective volume of the oximation reactor was 1.0-1.8 m³, the reaction temperature was 75-83°C and the circulation flow rate was 11.3 m³/h. The circulation pipeline was equipped with a metal membrane pipe filtration device (a cross-flow filter S1); after the reaction product 4 was filtered out clear liquid 5 through the cross-flow filter S1, the materials 6 were withdrawn at a flow rate of 1.7 m³/h into a cyclone extractor (the mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 900-1050 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 13, cyclohexanone (ketone 1), ammonia 3, and hydrogen peroxide 2 were respectively measured by a mass flow meter and then continuously added to an amidoximation reactor (a tank reactor R) containing a Ti-MOR catalyst. The flow rate of cyclohexanone (ketone 1) was 140-150 kg/h, the flow rate of hydrogen peroxide 2 was 180-210 kg/h with the concentration of 41.5%, and the flow rate of ammonia gas was 32-36 kg/h. The effective volume of the oximation reactor was 1.0-1.3 m³, the reaction temperature was 80-83°C and the circulation flow rate was 11.3-11.6 m³/h. The circulation pipeline was equipped with a metal membrane pipe filtration device (a cross-flow filter S1); after the reaction product 4 was filtered out clear liquid 5 through the cross-flow filter S1, the materials 6 were withdrawn at a flow rate of 1.4-1.8 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 930-1080 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 14, cyclohexanone (ketone 1), ammonia 3, and hydrogen peroxide 2 were respectively measured by a mass flow meter and then continuously added to an amidoximation reactor (a tank reactor R) containing a Ti-MOR catalyst. The flow rate of cyclohexanone (ketone 1) was 140-150 kg/h, the flow rate of hydrogen peroxide 2 was 180-210 kg/h with the concentration of 28-41.5%, and the flow rate of ammonia gas was 32-36 kg/h. The effective volume of the oximation reactor was 1.0-1.3 m³, the reaction temperature was 80-83°C and the circulation flow rate was 11.3-11.6 m³/h. The circulation pipeline was equipped with a metal membrane pipe filtration device (a cross-flow filter S1); after the reaction product 4 was filtered out clear liquid 5 through the cross-flow filter S1, the materials 6 were withdrawn at a flow rate of 1.4-1.8 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 930-1080 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled to the ammoximation reactor.

Embodiment 15, cyclohexanone (ketone 1), ammonia 3, and hydrogen peroxide 2 were respectively measured by a mass flow meter and then continuously added to an ammoximation reactor (a tank reactor R) containing a Ti-MOR catalyst. The flow rate of cyclohexanone (ketone 1) was 110-150 kg/h, the flow rate of hydrogen peroxide 2 was 120-210 kg/h with the concentration of 30-34.5%, and the flow rate of ammonia gas was 25-46 kg/h. The effective volume of the oximation reactor was 0.6-1.5 m³, the reaction temperature was 75-88°C and the circulation flow rate was 10.3-13.2 m³/h. The circulation pipeline was equipped with a metal membrane pipe filtration device (a cross-flow filter S1); after the reaction product 4 was filtered out clear liquid 5 through the cross-flow filter S1, the materials 6 were withdrawn at a flow rate of 1.3-1.5 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 920-1150 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

Embodiment 16, cyclohexanone (Ketone 1), ammonia 3, and Hydrogen Peroxide 2 were respectively measured by a mass flow meter and then continuously added to an ammoximation reactor (a tank Reactor R) containing a Ti-MOR catalyst. The flow rate of cyclohexanone (ketone 1) was 1100-1500 kg/h, the flow rate of hydrogen peroxide 2 was 1250-2200 kg/h with the concentration of 30-34.5%, and the flow rate of ammonia was 250-470 kg/h. The effective volume of the oximation reactor was 5-9.5 m³, the reaction temperature was 85-95°C and the circulation flow rate was 92-125 m³/h. The circulation pipeline was equipped with a metal membrane pipe filtration device (a cross-flow filter S1); after the reaction product 4 was filtered out clear liquid 5 through the cross-flow filter S1, the materials 6 were withdrawn at a flow rate of 12-16 m³/h into a cyclone extractor (a mixed extraction unit S2), and the rest was recycled back to the ammoximation reactor (the tank reactor R). Cyclohexane (an inert alkane solvent 9) was added to the cyclone extractor (the mixed extraction unit S2) at a flow rate of 8200-10900 kg/h, and the light phase (the organic phase 10) was separated out as a cyclohexane-oxime solution, which entered the subsequent rearrangement reactor. The heavy phase (the aqueous phase 11) separated from the cyclone extractor (the mixed extraction unit S2) was recycled back to the ammoximation reactor.

As shown in Fig. 4, a device for integrating ammoximation reaction and separation comprised a tank reactor R, a cross-flow filter S1 and a mixed extraction unit S2, wherein the tank reactor R, the cross-flow filter S1 and the mixed extraction S2 were successively connected, a ketone 1, ammonia 3 and hydrogen peroxide 2 were added to the tank reactor R for sufficient reaction, a reaction product 4 entered the cross-flow filter SI, clear liquid 5 in the cross-flow filter S1 was discharged, and the remaining substance was materials 6;

The discharge end of materials 6 of the cross-flow filter S1 was in communication with the tank reactor R via a material reflux pipe 13; in use, a portion of the materials 6 of the cross-flow filter S1 entered the mixed extraction unit S2, and the other portion of the materials 6 was refluxed to the tank reactor R via the material reflux pipe 13;

The discharge end of the aqueous phase 11 of the mixed extraction unit S2 was in communication with the tank reactor R via the aqueous phase reflux pipe 14, the organic phase 10 was discharged from the mixed extraction unit S2, and the aqueous phase 11 was refluxed to the tank reactor R via the aqueous phase reflux pipe 14;

The mixed extraction unit S2 was connected to an inert alkane solvent feed pipe 15, and the inert alkane solvent was added to the mixed extraction unit S2 through the inert alkane solvent feed pipe 15 .

The preferred embodiments of the present invention have been described above in detail, but the present invention is not limited to the specific details of the above-described embodiments. Within the scope of the inventive present invention, various simple modifications can be made to the technical solution, which all belong to the protection scope of the present invention.

## Claims

1. A method for integrating ammoximation reaction and separation, **characterized in that**, which comprises following steps:
(1) reacting a ketone, hydrogen peroxide and ammonia to form an oxime in the presence of a titanium silicon molecular sieve as a catalyst;
(2) separating clear liquid containing water and the oxime from reaction materials by cross-flow filtration;
(3) subjecting a portion of the materials after separation of the clear liquid to mixed extraction before being recycled back to a reactor, and adding an inert alkane solvent to extract so as to obtain an organic phase which is an oxime solution; and
(4) returning extracted aqueous phase turbid liquid containing the catalyst to an ammoximation reaction system.

2. The method for integrating ammoximation reaction and separation according to claim 1, **characterized in that**, in step (1), the titanium silicalite molecular sieve is one or more of Ti-MWW, TS-1, TS-2, Ti-β, Ti-SBA-15, Ti-MCM-41 or Ti-MOR.

3. The method for integrating ammoximation reaction and separation according to claim 1, **characterized in that**, in step (1), the ketone used is an aliphatic ketone with 3~10 carbon atoms, or a cycloaliphatic ketone or an aromatic ketone with 5~10 carbon atoms; a concentration of hydrogen peroxide used is 10%-80% by a mass fraction; and a molar ratio of the ketone, hydrogen peroxide to ammonia is 1: 1.0~2: 1.0~4.

4. The method for integrating ammoximation reaction and separation according to claim 1, **characterized in that**, in step (1), performing the ammoximation reaction under a condition of no solvent or a small amount of an inert alkane solvent, wherein reaction temperature is 40~100 °C, and reaction pressure is 0.1~1 MPa.

5. The method for integrating ammoximation reaction and separation according to claim 1, **characterized in that**, in step (2), controlling a liquid level of an ammoximation reactor to be constant by controlling a flow rate of the cross-flow filtration clear liquid, and a mass fraction of oxime in the cross-flow filtration clear liquid is 0.01%~50%.

6. The method for integrating ammoximation reaction and separation according to claim 1, **characterized in that**, in step (2), a cross-flow filtration equipment is a metal membrane pipe or a ceramic membrane pipe, and filtration accuracy is 20 nm~2000 nm.

7. The method for integrating ammoximation reaction and separation according to claim 1, **characterized in that**, in step (3), a ratio of a flow rate of materials to the mixed extraction to a flow rate of materials recycled back to the reactor is 0.01~0.3: 1.

8. The method for integrating ammoximation reaction and separation according to claim 1, **characterized in that**, in step (3), the inert alkane solvent is an alkane or cycloalkane or aromatic hydrocarbon with 4~10 carbon atoms or a mixture thereof which is insoluble or slightly soluble with water.

9. The method for integrating ammoximation reaction and separation according to claim 1, **characterized in that**, in step (3), a ratio of a flow rate of the inert alkane solvent to materials to the mixed extraction is (0.1~10): 1.

10. The method for integrating ammoximation reaction and separation according to claim 1, **characterized in that**, in step (4), a mass fraction of the catalyst in the aqueous phase turbid liquid returned to the ammoximation reaction system is 1%-10%.

11. An device for integrating ammoximation reaction and separation, **characterized in that**: the device comprises a tank reactor (R), a cross-flow filter (S1) and a mixed extraction unit (S2), and the tank reactor (R), the cross-flow filter (S1) and the mixed extraction unit (S2) are successively connected;
a discharge end of materials (6) of the cross-flow filter (S1) is in communication with the tank reactor (R) via a material reflux pipe (13);
a discharge end of aqueous phase (11) of the mixed extraction unit (S2) is in communication with the tank reactor (R) via an aqueous phase reflux pipe (14); and
the mixed extraction unit (S2) is connected to an inert alkane solvent feed pipe (15).
